# EUROPEAN PATENT APPLICATION

(11) **EP 1 136 552 A1**
(43) Date of publication of application: **26.09.2001**
(21) Application number: 00105820.5
(22) Date of filing: 20.03.2000
(51) Int. Cl.: C12N 15/12, C12N 15/11, C12N 9/00, C12N 5/10, C07K 14/705, C07K 16/28, C12Q 1/68, G01N 33/53, A61K 48/00

(54) **ATP binding cassette transporter 1 (ABC1) gene polymorphisms and uses thereof for the diagnosis and treatment of lipid disorders, cardiovascular diseases and inflammatory diseases**

(71) Applicant: Bayer Aktiengesellschaft, 51368 Leverkusen (DE)
(72) Inventor: Schmitz, Gerd, Prof.Dr., 93161 Sinzig (DE); Bodzioch, Marek, Dr., 30-601 Krakow (PL)

(57) **Abstract**

The present invention provides four common polymorphisms in the gene encoding the ATP-binding cassette transporter-1 (*ABC1*), which are associated with decreased ApoA-I mediated efflux of cholesterol, the first step of reverse cholesterol transport. The provided data can be taken as evidence that common polymorphisms in *ABC1* directly affect cellular lipid homeostasis, which is a key factor in the atherogenetic process. The frequencies of three of the common *ABC1* variants are significantly increased in a population of men having low HDL-C levels and established CHD relative to CHD-free control subjects. The use of the provided ABC1 polymorphisms for the diagnosis and treatment of lipid disorders, cardiovascular diseases, and inflammatory diseases like psoriasis and lupus erythematodes is claimed.

## Description

### Background of the invention

Epidemiological studies have established that plasma high density lipoprotein cholesterol (HDL-C) concentrations are inversely associated with the incidence of coronary heart disease (CHD) (1-4). Moreover, HDL deficiency is the most common lipid abnormality observed in patients with premature CHD (5,6), with nearly half of all CHD patients having low concentrations of HDL-C. Genetic factors account for approximately 50% of the variation in HDL-C levels in the general population (7), with several common mutations identified in genes encoding for proteins involved in the regulation of plasma HDL-C concentrations and, ultimately, playing a role in the development of CHD (8).

Recently, we (9,10) and others (11-15) demonstrated that familial HDL deficiency (FDH) syndromes with either splenomegalie or atherosclerosis (e.g. Tangier disease (TD); familial hypoalphaglycoproteinaemia) are rare HDL-deficiencies caused by mutations in the gene encoding the ATP-binding cassette transporter-1 (ABC1). ABC1 is a member of the ATP binding cassette family of proteins that are involved in the transmembrane transport of a variety of different molecules, including bile acids, steroid hormones, ions, amino acids, sugars and vitamins (16-19). TD is characterized by severely diminished plasma HDL-C concentrations, reduced levels of low density lipoprotein cholesterol (LDL-C), the deposition of cholesteryl esters in the reticulo-endothelial system with splenomegaly and enlargement of tonsils and lymph nodes (20), as well as a predisposition to neuropathy and CHD (21,22). The HDL deficiency in these patients has been related to a rapid catabolism of HDL and a decrease in ApoA-I induced lipid efflux from various cells including mononuclear phagocytes (23-25). Familial hypoalphaglycoproteinaemia represents the other clinical phenotype that is characterized by a similar disturbance in ApoA-I metabolism but is associated with an enhanced risk of CHD rather than splenomegalie. ApoA-I mediated cellular cholesterol efflux is the first step in the reverse cholesterol transport and prevents excessive cholesterol accumulation in peripheral cells. In addition, this process is suggested to contribute to the formation of mature HDL particles, thereby influencing the pool size of HDL in the plasma.

Although mutations in *ABC1* cause TD, the precise molecular mechanism by which they do so remains unknown. Also, little is known about the association of ABC1 and HDL metabolism in individuals not affected with TD. Previous investigations on the function of ABC1 in mice have revealed its role in the engulfment of apoptotic cells by macrophages (26) and in macrophage interleukin-1β secretion (27). We have further shown that ABC1 is a cholesterol-responsive modulator of cellular lipid efflux (10) and, thus, plays a significant role in cholesterol homeostasis. Moreover, the ABC1-knockout mice (ABC1-/-) show massively reduced levels of serum lipids and lipoproteins. The expression of ABC1 in mucosa cells of the small intestine and the altered lipoprotein metabolism in ABC1-/- mice allows the conclusion that ABC1 plays also a major role in intestinal resorption and translocation of lipids into the lymph-system.

### Summary of the invention

Four different polymorphisms in *ABC1* (G596A, T1136C, A2589G, and G3456C) were identified in different Tangier kindreds as well as 516 healthy control subjects. Each of these polymorphisms affect the primary structure of the gene product.

A frequent coincidence of the G596A and A2589G polymorphisms was identified indicating that those two mutations are in linkage disequilibrium.

The two most common polymorphisms (G596A and A2589G) are both associated with a decreased in vitro ApoA-I mediated efflux of cholesterol from mononuclear phagocytes emphasizing the role of ABC1 in lipid efflux, the first step in reverse cholesterol transport. These results also are evidence that common polymorphisms in *ABC1* directly affect cellular lipid homeostasis, which is a key factor in the atherogenic process. A reduction of ApoA-I mediated efflux of cholesterol from mononuclear phagocytes is also a typical feature of Tangier disease.

Plasma lipoproteins, and HDL-cholesterol in particular, are not affected, probably because the effect of the *ABC1* polymorphisms on the protein function is compensated by other factors controlling plasma HDL levels.

Three common variants in the gene encoding ABC1 (G596A, A2589G, and G3456C) occur with significantly increased frequency in a population of men having low HDL-C as their primary lipid abnormality and established CHD, providing evidence for a role of *ABC1* in the premature CHD associated with common HDL deficiency states.

In summary, the identified polymorphisms in the ABC1 gene can be used for diagnosis and therapy of lipid disorders, cardiovascular diseases, and inflammatory diseases.

### Abbreviations

| | |
|---|---|
| ABC | ATP-binding cassette |
| ANC | Anchor |
| ApoA | Apolipoprotein A |
| ApoB | Apolipoprotein B |
| ApoE | Apolipoprotein E |
| ATP | Adenosine triphosphate |
| BMI | Body mass index |
| bp | Base pairs |
| cAMP | Cyclic adenosin monophosphat |
| cDNA | Copy DNA |
| CH | Cholesterol |
| CHD | Coronary heart disease |
| DET | Detection |
| DPM | Disintegrations per minute |
| E-LDL | Enzymatically modified LDL |
| f | Forward |
| Fluo | Fluorescein |
| FOS | Framingham Offspring Study |
| HDL | High density lipoprotein |
| HDL-C | HDL-cholesterol |
| kDa | Kilo Dalton |
| LDL | Low density lipoprotein |
| LDL-C | LDL-cholesterol |
| M-CSF | Macrophage colony stimulating factor |
| ph | Phosphorylation |
| r | Reverse |
| SD | Standard seviation |
| SDS | Sodium dodecyl sulfate |
| TD | Tangier Disease |
| TG | Triglycerides |
| VA-HIT | Veterans Affairs Cooperative HDL Cholesterol Intervention Trial |
| wt | Wild type |

### 1. References cited

1. Gordon, T., Castelli, W.P., Hjortland, M.C., Kannel, W.B., Dawber, T.R. High density lipoprotein as a protective factor against coronary heart disease. The Framingham Study. *Am. J. Med.* 1977; **62:** 707-14.
2. Kannel, W.B. High density lipoproteins: epidemiologic profile and risks of coronary artery disease. *Am. J. Cardiol.* 1983; **52:** 98-128.
3. Goldbourt, U., Yaari, S., Medalie, J.H. Isolated low HDL cholesterol as a risk factor for coronary heart disease mortality: a 21-year follow-up of 8000 men. *Arterioscler. Thromb. Vasc. Biol.* 1997; **17**: 107-13.
4. Wilson, P.W.F., D'Agostino, R.B., Levy, D., Belanger, A.M., Silbershatz, H., Kannel, W.B. Prediction of coronary heart disease using risk factor categories. *Circulation.* 1998; **97**: 1837-47.
5. Genest, J., Jr., et al. Familial lipoprotein disorders in patients with premature coronary artery disease. *Circulation* 1992; **85**: 2025-33.
6. Genest, J., Jr., Bard, J.-M., Fruchart, J.-C., Ordovas, J.M., Schaefer, E.J. Familial hypoalphalipoproteinemia in premature coronary artery disease. *Arterioscler. Thromb.* 1993; **13**: 1728-37.
7. Heller, D.A., DeFaire, D., Pedersen, N.L., Dahlen, G. , McClearn, G.E. Genetic and environmental influences on serum lipid levels in twins. *N. Engl. J Med.* 1993; **328**: 1150-56.
8. Calabresi, L., Franceschini, G. High density lipoproteins and coronary heart disease: insights from mutations leading to low high density lipoprotein. *Curr. Opin. Lipidol.* 1997; **8**: 219-24.
9. Bodzioch, M., et al. The gene encoding ATP-binding cassette transporter 1 is mutated in Tangier disease. *Nat. Genet.* 1999; **22**: 347-51.
10. Langmann T, Klucken J, Reil M, Liebisch G, Luciani MF, Chimini G, Kaminski WE, Schmitz G. Molecular cloning of the human ATP-binding cassette transporter 1 (hABC1): evidence for sterol-dependent regulation in macrophages. *Biochem Biophys Res Commun* 1999; **257**:29-33
11. Brooks-Wilson, A., et al. Mutations in *ABC1* in Tangier disease and familial high-density lipoprotein deficiency. *Nat. Genet.* 1999; **22**: 336-45.
12. Rust, S., et al. Tangier disease is caused by mutations in the gene encoding ATP-binding cassette transporter 1. *Nat Genet.* 1999; **22**: 352-55.
13. Remaley, A.T., et al. Human ATP-binding cassette transporter 1 (*ABC1):* Genomic organization and identification of the genetic defect in the original Tangier disease kindred. *Proc. Natl. Acad. Sci.* 1999; **96**: 12685-90.
14. Lawn, R.M., et al. The Tangier disease gene product ABC1 controls the cellular apolipoprotein-mediated lipid removal pathway. *J. Clin. Invest.* 1999; **104**: R25-R31.
15. Brousseau, M.E., et al. Novel mutations in the gene encoding ATP-binding cassette 1 in four Tangier disease kindreds. *J. Lipid Res.* (in press).
16. Higgins CF. ABC transporters: from microorganisms to man. *Annu Rev Cell Biol* 1992; **8**:67-113
17. Higgins CF. Flip-flop: the transmembrane translocation of lipids. *Cell* 1994; **79**:393-5
18. van Helvoort A, Smith AJ, Sprong H, Fritzsche I, Schinkel AH, Borst P. van Meer G. MDR1 P-glycoprotein is a lipid translocase of broad specificity, while MDR3 P-glycoprotein specifically translocates phosphatidylcholine. *Cell* 1996; **87**:507-17
19. Dekkers DW, Comfurius P, Schroit AJ, Bevers EM, Zwaal RF. Transbilayer movement of NBD-labeled phospholipids in red blood cell membranes: outward-directed transport by the multidrug resistance protein 1 (MRP1). *Biochemistry* 1998; **37**:14833-7
20. Assmann G, Schmitz G & Brewer HB Jr. Familial high density lipoprotein deficiency: Tangier disease. In: Scriver C.R, Beaudet, AL, Sly, WS, Valle, D (eds): The metabolic basis of inherited disease. 6^{th} ed., McGraw-Hill, New York Chapt 1989; **50**: 1267-1282.
21. Assmann, G., von Eckardstein, A., Brewer, H.B., Jr. Familial high density lipoprotein deficiency: Tangier disease. *In The Metabolic and Molecular Basis of Inherited Disease* (eds Scriver, C.R. et al.) 2053-2072 (McGraw-Hill, New York, 1995).
22. Serfaty-Lacrosniere, C., et al. Homozygous Tangier disease and cardiovascular disease. *Atherosclerosis* 1994; **107**: 85-98.
23. Schmitz G, Assmann G, Robenek H & Brennhausen B. Tangier disease: A disorder of intracellular membrane traffic. *Proc. Natl. Acad. Sci. USA* 1985; **82**: 6305-6309.
24. ^{Rogler} G, Trümbach B, Klima B, Lackner KJ & Schmitz G High density lipoprotein-mediated efflux of newly synthesized cholesterol is impaired in fibroblasts from Tangier patients while membrane description and efflux of lysosomal cholesterol are normal. *Arterioscler. Thromb. Vasc. Biol.* 1995; **15**: 683-690.
25. Francis GA, Knopp RH & Oram JF Defective removal of cellular cholesterol and phospholipids by apolipoprotein A-I in Tangier Disease. *J*. *Clin. Invest.* 1995; **96**: 78-87.
26. Luciani, M.F., Chimini, G. The ATP binding cassette transporter ABC1 is required for engulfment of corpses generated by apoptotic cell death. *EMBO J.* 1996; **15**: 226-35.
27. Hamon, Y., et al. Interleukin-1β secretion is impaired by inhibitors of the ATP binding cassette transporter, ABC1. *Blood* 1997; **90**: 2911-15.
28. Sakr SW, Williams DL, Stoudt GW, Phillips MC & Rothblat GH. Induction of cellular cholesterol efflux to lipid-free apolipoprotein A-I by cAMP. Biochim. Biophys. Acta 1999; **19**:1438:85-98.
29. Aslanidis C & Schmitz G. High speed apolipoprotein E genotyping and apolipoprotein B3500 mutation detection using real-time fluorescence PCR and melting curves. *Clin. Chem.* 1999; **45**: 1094-1097.
30. Aslanidis C, Nauck M & Schmitz G. High-speed prothrombin G A 20210 and methylenetetrahydrofolate reductase C T 677 mutation detection using real-time fluorescence PCR and melting curves. *Biotechniques* 1999; **27**: 234-236.
31. Bhakdi S, Dorweiler B, Kirchmann R, Torzewski J, Weise E, Tranum-Jensen J, Walev I, Wieland E. On the pathogenesis of atherosclerosis: enzymatic transformation of human low density lipoprotein to an atherogenic moiety. *J*. *Exp. Med.* 1995; **182**: 1959-1971.
32. Feinleib, M., Kannel, W.B., Garrison, R.J., McNamara, P.M., Castelli, W.P. The Framingham Offspring Study. Design and preliminary data. *Prev. Med.* 1975; **4**: *518-25.*
33. Rubins, H.B., et al. Gemfibrozil for the secondary prevention of coronary heart disease in men with low levels of high density lipoprotein cholesterol. *N. Engl. J. Med.* 1999; **341**: 410-18.
34. Wittwer, C.T., Ririe, K.M., Andrew, R.V., David, D.A., Gundry, R.A., Balis, U.J. The LightCycler: A microvolume multisample fluorimeter with rapid temperature control. *BioTechniques* 1997; **22**: 176-81.
35. Reiser, A., Geyer, M., van Miltenburg, R., Nauck, M., Tabiti, K. Mutation detection using multi-color detection on the LightCycler system. *Biochemica* 1999; **2**: 12-15.
36. Schaefer, E.J., et al. Factors associated with low and elevated plasma high density lipoprotein cholesterol and apolipoprotein A-I levels in the Framingham Offspring Study. *J*. *Lipid Res.* 1994; **35**: 871-82.
37. McNamara, J.R., Schaefer, E.J. Automated enzymatic standardized lipid analyses for plasma and lipoprotein fractions. *Clin. Chim. Acta.* 1987; **166**: 1-8.
38. Warnick, G.R., Benderson, J., Albers, J.J. Dextran sulfate-Mg2+ precipitation procedure for quantitation of high-density lipoprotein cholesterol. *Clin. Chem.* 1982; **28**: 1379-88.
39. Friedewald, W.T., Levy, R.I., Fredrickson, D.S. Estimation of the concentration of low-density lipoprotein cholesterol in plasma, without use of the preparative ultracentrifuge. *Clin. Chem.* 1972; **18**: 499-502.
40. Orso, E., Broccardo, C., Kaminski, W.E., Bottcher, A., Liebisch, G., Drobnik, W., Gotz, A., Chambenoit, O., Diederich, W., Langmann, T., Spruss, T., Luciani, M.F., Rothe, G., Lackner, K.J., Chimini, G. Schmitz, G. Transport of lipids from golgi to plasma membrane is defective in tangier disease patients and Abc1-deficient mice. *Nat. Genet.* 2000, **24**:192-196
41. Smythe, J.A., Symonds, G. Gene therapeutic agents: The use of ribozymes, antisense, and RNA decoys for HIV-1 infection. *Inflamm. Res.* 1995, **44**:11-15

### Description of Tables:

### Table 1:

**Summary of the characteristics of the study population consisting of 516 healthy blood donors.** Data are expressed as mean ± SD. n indicates the number of analyzed individuals.

### Table 2:

**Frequency of *ABC1* polymorphisms G596A, A2589G, G3456C, and T1136C in healthy control subjects.** Genotyping was performed by LightCycler (Roche Diagnostics) PCR analysis. Data provide the relative percentage of homozygous, heterozygous or wild type individuals at the different polymorphic sites. n indicates the number of analyzed individuals.

### Table 3:

**Distribution of G596A and A2589G polymorphisms in healthy control subjects.** Data represent absolute numbers of subjects with the respective allele combination. Data in parenthesis provide the relative percentage within each group.

### Table 4:

**Amplification primers and hybridization probes for the LightCycler polymorphism analysis.** Hybridization probes are designated as detection (DET) or anchor (ANC) probes. As indicated, they are labeled with LightCycler-Red 640 or 705 and fluorescein, respectively. Phosphorylation (ph) of the 3'-end prevents the elongation of the detection probe. All detection probes recognize wild-type sequences, with the exception of the probe DET1136/mut. Fluo - fluorescein.

### Table 5:

**Distribution of lipid and lipoprotein values in healthy individuals with different *ABC1* polymorphisms.** HDL-C and ApoA-I levels in homozygous, heterozygous, and wild type male and female individuals are presented. Data are expressed as mean ± SD. n indicates the number of analyzed individuals.

### Table 6:

**Cholesterol and phopholipid efflux from mononuclear phagocytes isolated form healthy individuals homozygous for the wild type nucleotide at all four polymporphic sites and from healthy individuals homozygous for - the polymorphic allele at position 596 or 2589.** Lipid efflux is expressed as percent of basal unstimulated efflux observed in serum-free macrophage medium without ApoA-I. Data are the mean ± SD of the indicated number (n) of different subjects. Efflux analysis for each subject was performed at least in quadruplicate. P values were calculated by independent Student T-test for wt/wt versus G596A and A2589G, respectively.

### Table 7:

**Characteristics of *FOS* and VA-HIT subjects.** Results are listed as means±SD. ^{*}Significantly different from male FOS control subjects, P<0.01.

### Table 8:

**Genotype distribution of the *ABC1* polymorphisms in FOS versus VA-HIT** subjects. Values in parentheses are expressed as a percentage of the total number of subjects within each group.

### Table 9:

**Association of the G596A and A2589G *ABC1* polymorphisms in FOS and VA-HIT subjects.** Data represent absolute numbers of subjects out of a total of 1,008 for FOS and 1,014 for VA-HIT, whereas values in parentheses (%) provide the relative percentage of subjects within each group (FOS or VA-HIT). Significant differences in proportions between FOS and VA-HIT are presented in bold face type.

### Table 10:

**Association of the G596A and G3456C *ABC1* polymorphisms in FOS and VA-HIT subjects.** Data represent absolute numbers of subjects out of a total of 1,006 for FOS and 1,014 for VA-HIT, whereas values in parentheses (%) provide the relative percentage of subjects within each group (FOS or VA-HIT). Significant differences in proportions between FOS and VA-HIT are presented in bold face type.

### Table 11:

**Association of the A2589G and G3456C *ABC1* polymorphisms in FOS and VA-HIT subjects.** Data represent absolute numbers of subjects out of a total of 1,008 for FOS and 1,014 for VA-HIT, whereas values in parentheses (%) provide the relative percentage of subjects within each group (FOS or VA-HIT). Significant differences in proportions between FOS and VA-HIT are presented in bold face type.

**Table 1:**

| **Summary of the characteristics of the study population consisting of 516 healthy blood donors.** | | | | | | |
|---|---|---|---|---|---|---|
| | n | Age | Cholesterol mmol/l | Triglycerides mmol/l | HDL-C mmol/l | LDL-C mmol/l |
| Men | 274 | 27.3 ± 7.7 | 4.95 ± 1.13 | 1.23 ± 0.85 | 1.43 ± 0.35 | 2.97 ± 1.01 |
| Women | 242 | 30.5 ± 10.2 | 5.63 ± 1.10 | 1.19 ± 0.56 | 1.72 ±0.45 | 3.24 ± 1.02 |

**Table 2:**

| **Frequency of ABC1 Polymorphisms in healthy control subjects** | | | | |
|---|---|---|---|---|
| | **n** | **Homozygous** | **Heterozygous** | **Wild Type** |
| **G596A** | 511 | 8.0 % | 43.5 % | 48.5 % |
| **A2589G** | 514 | 2.3 % | 22.8 % | 74.9 % |
| **G3456C** | 516 | 0.0 % | 5.0 % | 95.0 % |
| **T1136C** | 515 | 0.0 % | 0.6 % | 99.4 % |

**Table 3:**

| **Distribution of G596A and A2589G Polymorphisms in healthy control subjects** | | | |
|---|---|---|---|
| | **G596A** | | |
| | 596A/A | 596G/A | 596G/G |
| **A2589G** | | | |
| 2589G/G | 4 (9.8%) | 6 (2.7%) | 2 (0.8%) |
| 2589A/G | 14 (34.1%) | 61 (27.6%) | 39 (15.7%) |
| 2589A/A | 23 (56.1%) | 154 (69.7%) | 207 (83.5%) |

**Table 4:**

| **Amplification primers and hybridization probes for the LightCycler polymorphism analysis** | |
|---|---|
| G596A | |
| DET596/705 | 5'-LC Red 705-GCCTACCAAGGGAGAAACTG-ph |
| ANC596 | 5'-CTTGGTGACCAAGAAGTTTCTGAGCTTTG-Fluo |
| FLA596-f | 5'-TTTTGCAAGGCTACCAGTTACA |
| FLA596-r | 5'-CAGGATTGGCTTCAGGATGT |

| T1136C | |
|---|---|
| DET1136/mut | 5'-LC Red 640-ACTCCAGGCGAACAAGACC-ph |
| ANC1136/B | 5'-TTTCTGCGGTCCCTGGCTCCCCACC-Fluo |
| FLA1136-f | 5'-AAGTCTGATGCAGACCAGAGC |
| FLA1136-r | 5'-CGGACAAGGTCCATTTCTTG |

| A2589G | |
|---|---|
| DET2589rev | 5'-LC Red 640-CTTTCTGATATTCTCTTCTGG-ph |
| ANC2589rev | 5'-TTAAAGAAAGAGCAGGAGGTCAACAGCACT-Fluo |
| FLA2589-f | 5'-AGGCCCTGGTATTTTCCTTG |
| FLA2589-r | 5'-CCCTGGAGTGGTTTCACAGT |

| G3456C | |
|---|---|
| DET3456rev | 5'-LC Red 640-CGTGTCACTCTCATGGTCG-ph |
| ANC3456rev | 5'-AGAAACCCCAGAGTCCTTACCGATGGTC-Fluo |
| FLA3456-f | 5'-GGACAGTGTTTCTCAGAGCAG |
| FLA3456-r | 5'-AGCAGCAAACCTTGAGTCAG |

**Table 5:**

| **Distribution of lipid and lipoprotein values in healthy individuals with different *ABC1* polymorphisms** | | | |
|---|---|---|---|
| | Homozygous | Heterozygous | Wild Type |
| **G596A** | *596A/A* | *596G/A* | *596G/G* |
| ***men*** | n=19 | n=127 | n=126 |
| HDL-C (mmol/l) | 1.41 ± 0.26 | 1.45 ± 0.36 | 1.41 ± 0.34 |
| ApoA-I(g/l) | 1.51 ± 1.6 | 1.49 ± 2.5 | 1.48 ± 2.3 |
| ***women*** | n=22 | n=95 | n=122 |
| HDL-C (mmol/l) | 1.82 ± 0.45 | 1:75 ± 0.46 | 1.73 ± 0.44 |
| ApoA-I(g/l) | 1.77 ± 3.9 | 1.79 ± 3.6 | 1.76 ± 3.3 |

| A2589G | *2589G/G* | *2589A/G* | *2589A/A* |
|---|---|---|---|
| ***men*** | n=7 | n=54 | n=212 |
| HDL-C (mmol/l) | 1.50 ± 0.28 | 1.39 ± 0.32 | 1.44 ± 0.36 |
| ApoA-I(g/l) | 1.62 ± 21 | 1.47 ± 24 | 1.49 ± 24 |
| ***women*** | n=5 | n=63 | n=173 |
| HDL-C (mmol/l) | 1.94 ± 0.40 | 1.80 ± 0.37 | 1.72 ± 0.48 |
| ApoA-I(g/l) | 2.02 ± 28 | 1.79 ± 32 | 1.76 ± 36 |

| **G3456C** | *3456C/C* | *3456G/C* | *3456G/G* |
|---|---|---|---|
| ***men*** | n=0 | n=15 | n=228 |
| HDL-C (mmol/l) | | 1.37 ± 0.27 | 1.44 ± 0.35 |
| ApoA-I(g/l) | | 1.50 ± 26 | 1.49 ± 24 |
| ***women*** | n=0 | n=11 | n=262 |
| HDL-C (mmol/l) | | 1.84 ± 0.46 | 1.74 ± 0.45 |
| ApoA-I (g/l) | | 1.79 ± 34 | 1.77 ± 35 |

**Table 6:**

| **Cholesterol and phopholipid efflux from mononuclear phagocytes isolated form healthy individuals homozygous for the wild type nucleotide at all four polymporphic sites and from healthy individuals homozygous for the polymorphic allele at position 596 or 2589.** | | | |
|---|---|---|---|
| **A. Cholesterol efflux: ApoA-I [10 µg/ml]** | | | |
| | **wt/wt 596G/G; 2589A/A** | **596A/A** | **2589G/G** |
| *all probands* | 128.7 ± 17.5% | 97.7 ± 22.4% | 103.4 ± 18.7% |
| | (n=18) | (n=8; p<0.001) | (n=11; p<0.001) |
| *men* | 126.7 ± 16.6% | 98.8 ± 43.8% | 104.5 ± 23.0% |
| | (n=14) | (n=2; n.s.) | (n=6; p<0.025) |
| *women* | 135.6 ± 21.4% | 97.3 ± 18.0% | 102.0 ± 14.3% |
| | (n=4) | (n=6; p<0.001) | (n=5; p<0.025) |

| **B. Cholesterol efflux: ApoA-I [10 µg/ml]+ 24 h pretreatment with forskolin [10 µM]** | | | |
|---|---|---|---|
| | **wt/wt** | **596A/A** | **2589G/G** |
| | **596G/G; 2589A/A** | | |
| *men and women* | 121.8 ± 16.4% | 126.7 ± 37.4% | 121,5 ± 38.8% |
| | (n=12) | (n=8; n.s.) | (n=10; n.s.) |

| **C. Phospholipid efflux: ApoA-I [10 µg/ml]** | | | |
|---|---|---|---|
| | **wt/wt 596G/G; 2589A/A** | **596A/A** | **2589G/G** |
| *men and women* | 125.9 ± 15.3% | 117.3 ± 27.1% | 114,9 ± 32.6% |
| | (n=18) | (n=8; n.s.) | (n=10; n.s.) |

| **D. Phospholipid efflux: ApoA-I [10 µg/ml]+ 24 h pretreatment with forskolin [10 µM]** | | | |
|---|---|---|---|
| | **wt/wt 596G/G; 2589A/A** | **596A/A** | **2589G/G** |
| *men and women* | 135.4 ± 20.2% | 136.7 ± 12.8% | 124.5 ± 25.8% |
| | (n=12) | (n=8; n.s.) | (n=10; n.s.) |

**Table 7:**

| **Characteristics of FOS and VA-HIT subjects** | | |
|---|---|---|
| | **FOS** | **VA-HIT** |
| ***N*** | 1014 | 1014 |
| **Age (years)** | 53±9 | 64±7* |
| **BMI (kg/m**^{**2**}**)** | 27.7±3.9 | 29±5* |
| **TC (mg/dL)** | 206±36 | 175±25* |
| **LDL-C (mg/dL)** | 136±33 | 111±23* |
| **HDL-C (mg/dL)** | 44±12 | 32±5* |
| **TG (mg/dL)** | 138±101 | 161±68 |
| **TC:HDL-C** | 5.1±1.6 | 5.7±1.1* |

| | | |
|---|---|---|
| *Significantly different from male FOS control subjects, P<0.01. | | |

**Table 8:**

| **Genotype distribution of the *ABC1* polymorphisms in FOS versus VA-HIT subjects** | | | |
|---|---|---|---|
| **Mutation** | **FOS** | **VA-HIT** | **P** |
| **G596A** | | | |
| Total n | 1013 | 1014 | |
| GG (%)* | 543 (53.6) | 454 (44.8) | |
| GA (%) | 402 (39.7) | 484 (47.7) | |
| AA (%) | 68 (6.7) | 76 (7.5) | |
| **A Allele frequency** | **0.266** | **0.314** | **0.000** |
| | | | |

| **A2589G** | | | |
|---|---|---|---|
| Total n | 1014 | 1014 | |
| AA (%) | 779 (76.8) | 707 (69.7) | |
| AG (%) | 222 (21.9) | 285 (28.1) | |
| GG (%) | 13 (1.3) | 22 (2.2) | |
| G Allele frequency | 0.122 | 0.162 | 0.001 |
| | | | |

| G3456C | | | |
|---|---|---|---|
| **Total *n*** | 1013 | 1014 | |
| GG (%) | 965 (95.3) | 940 (92.7) | |
| GC (%) | 43 (4.2) | 70 (6.9) | |
| CC (%) | 5 (0.5) | 4 (0.4) | |
| C Allele frequency | 0.026 | 0.038 | 0.032 |

**Table 9:**

| **Association of the G596A and A2589G *ABC1* polymorphisms in FOS and VA-HIT subjects** | | | | | | |
|---|---|---|---|---|---|---|
| | | | **G596A** | | | |
| | ***GG*** | | **GA** | | **AA** | |
| | FOS | **VA-HIT** | FOS | VA-HIT | FOS | VA-HIT |
| A2589G | | | | | | |
| **AA** | 459 | 360 | 77 | 89 | 5 | 5 |
| | **(45.6)** | **(35.5)** | (7.6) | (8.8) | (0.5) | (0.5) |
| **AG** | 268 | 30 | 125 | 166 | 5 | 12 |
| | **(26.6)** | **(30.2)** | **(12.4)** | **(16.4)** | (0.5) | (1.2) |
| **GG** | 47 | 41 | 18 | 30 | 3 | 5 |
| | (4.7) | (4.0) | **(1.8)** | **(3.0)** | (0.3) | (0.5) |

**Table 10:**

| **Association of the G596A and G3456C *ABC1* polymorphisms in FOS and VA-HIT subjects** | | | | | | |
|---|---|---|---|---|---|---|
| **G596A** | | | | | | |
| | ***GG*** | | **GA** | | **AA** | |
| | FOS | VA-HIT | FOS | VA-HIT | FOS | VA-HIT |
| **G3456C** | | | | | | |
| **GG** | 508 | 426 | 27 | 26 | 3 | 2 |
| | (50.5) | (42.0) | (2.7) | (2.6) | (0.3) | (0.2) |
| **GC** | 384 | 450 | 14 | 34 | 2 | 0 |
| | **(38.2)** | **(44.4)** | **(1.4)** | **(3.4)** | (0.2) | (0) |
| **CC** | 66 | 64 | 2 | 10 | 0 | 2 |
| | (6.6) | (6.3) | (0.2) | (1.0) | (0) | (0.2) |

**Table 11:**

| **Association of the A2589G and G3456C *ABC1* polymorphisms in FOS and VA-HIT subjects** | | | | | | |
|---|---|---|---|---|---|---|
| **A2589G** | | | | | | |
| | ***AA*** | | **AG** | | **GG** | |
| **G3456C** | | | | | | |
| **GG** | 746 | 666 | 204 | 257 | 11 | 17 |
| | **(74.0)** | **(65.7)** | **(20.2)** | **(25.3)** | (1.1) | (1.7) |
| **GC** | 26 | 39 | 15 | 27 | 1 | 4 |
| | (2.6) | (3.8) | **(1.5)** | **(2.7)** | (0.1) | (0.4) |
| **CC** | 3 | 2 | 2 | 1 | 0 | 1 |
| | (0.3) | (0.2) | (0.2) | (0.1) | (0) | (0.1) |

### Description of specific embodiments

### Identification of four polymorphisms in the gene encoding the human ATP-binding cassette transporter 1 (ABC1) and association of these polymorphisms with decreased ApoA-I mediated efflux of cholesterol

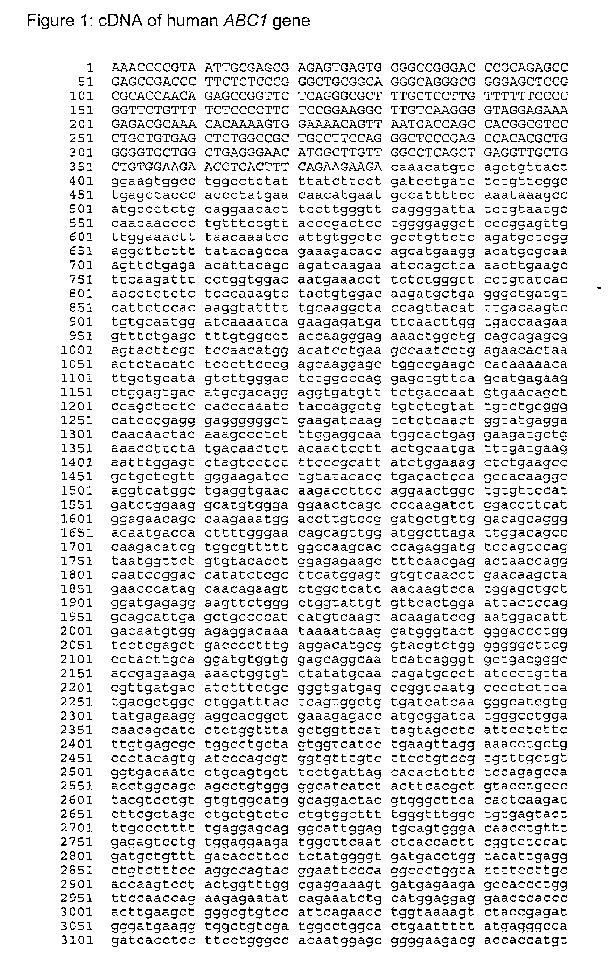

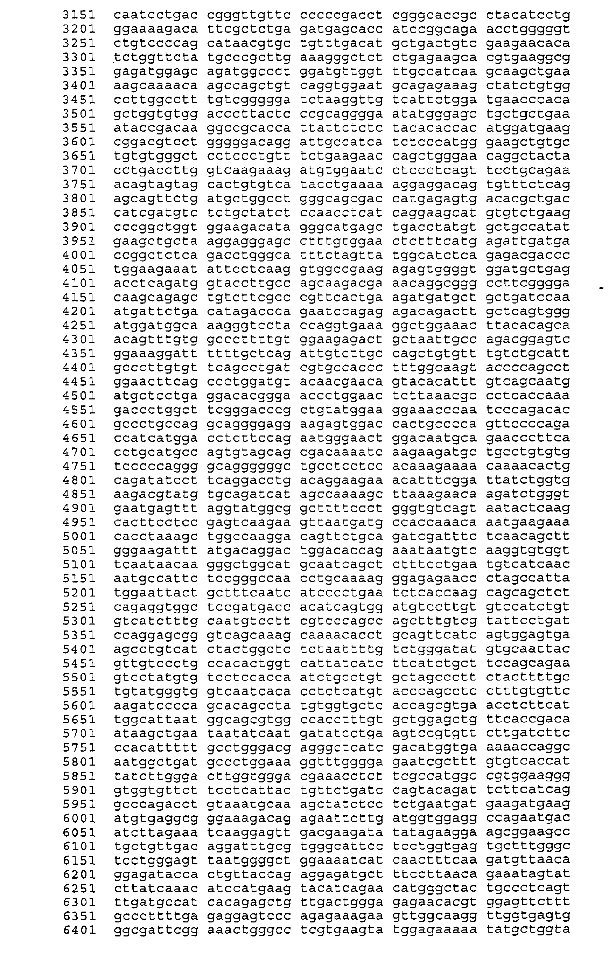

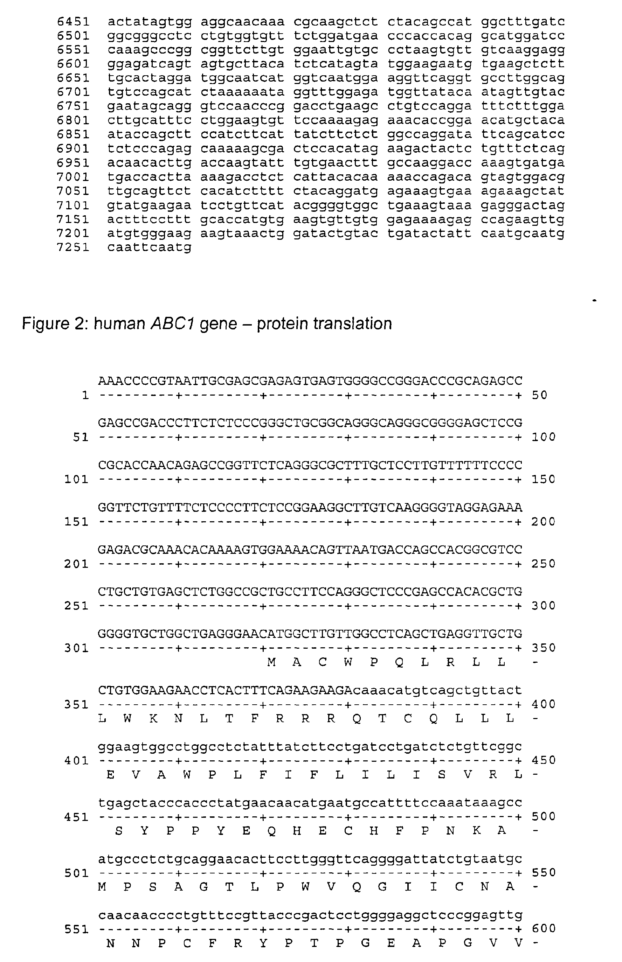

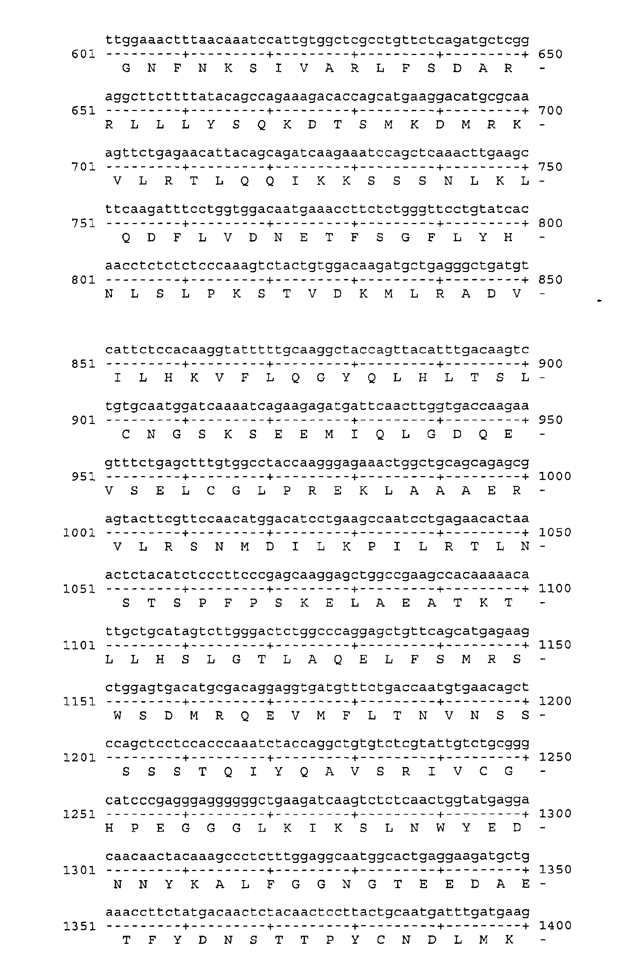

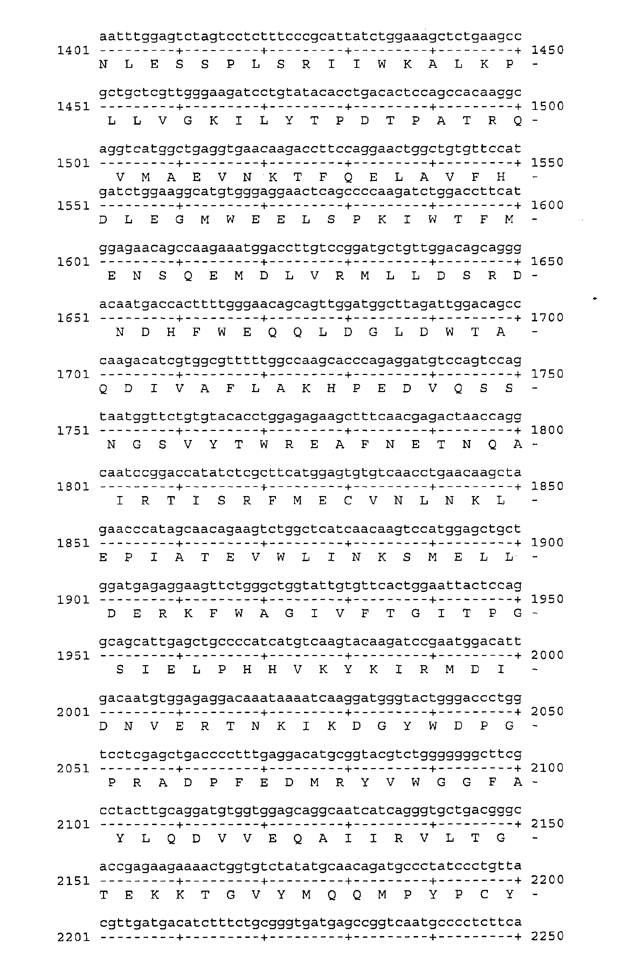

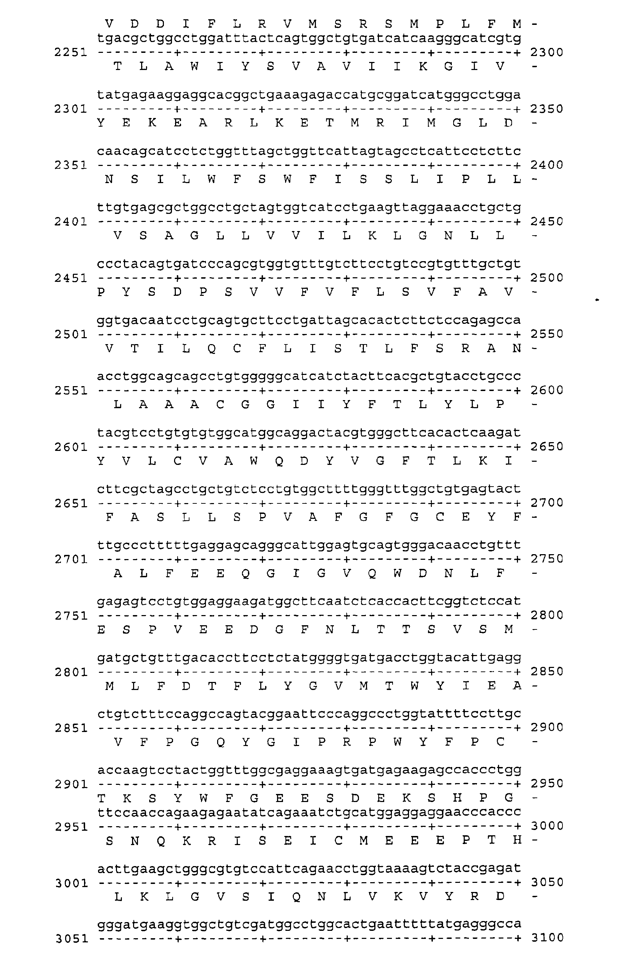

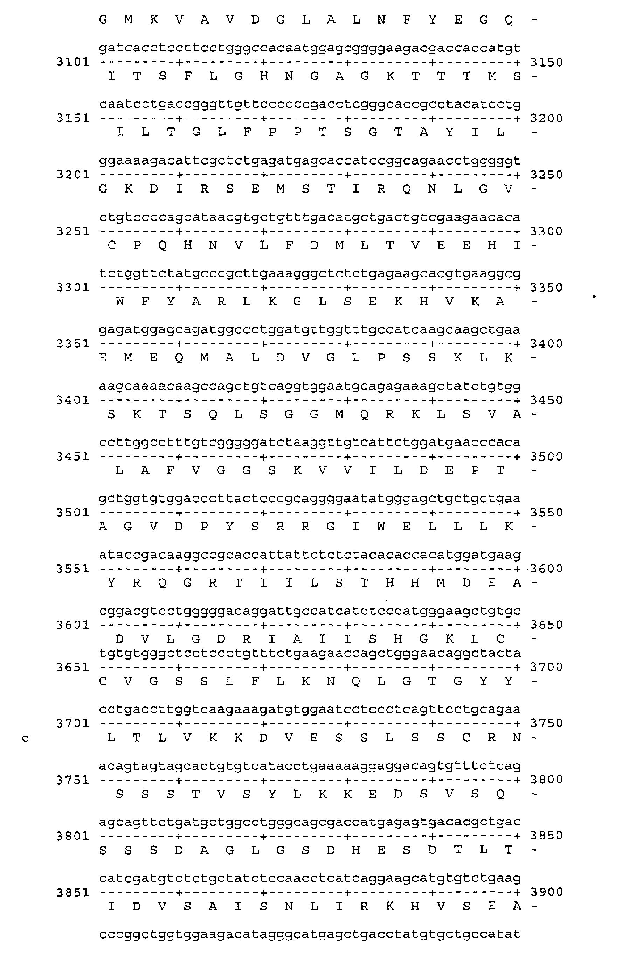

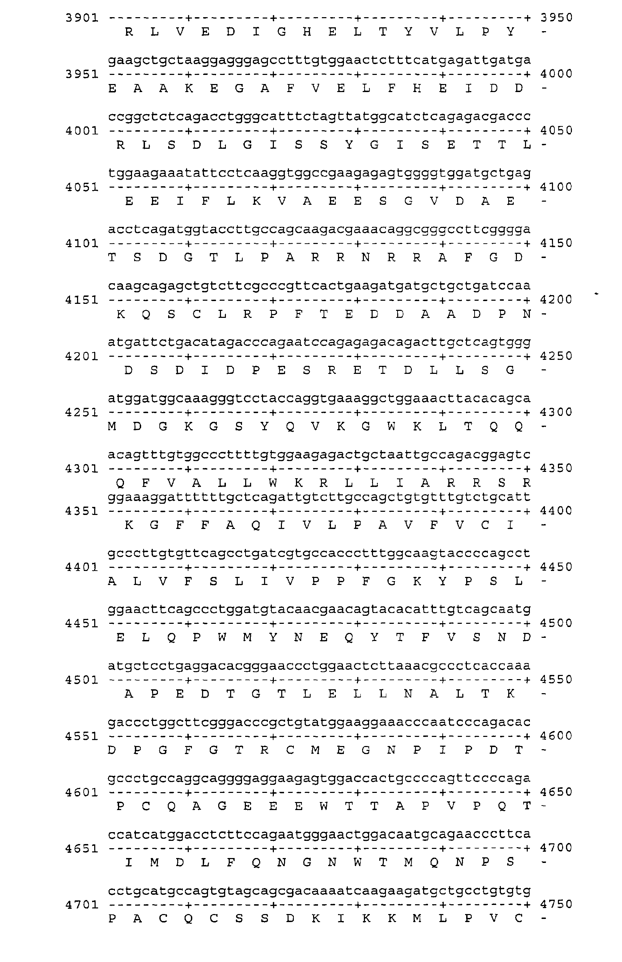

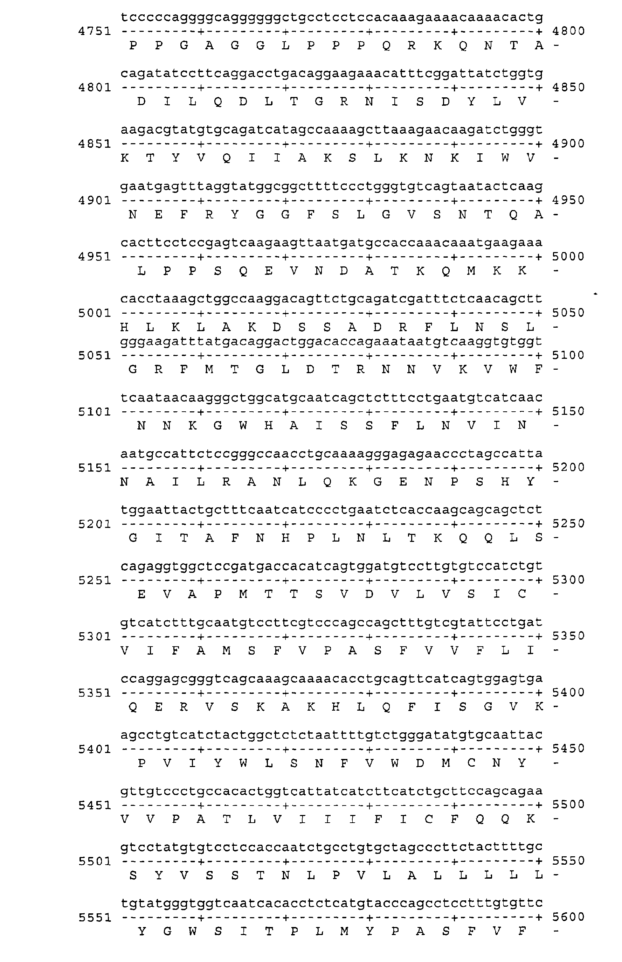

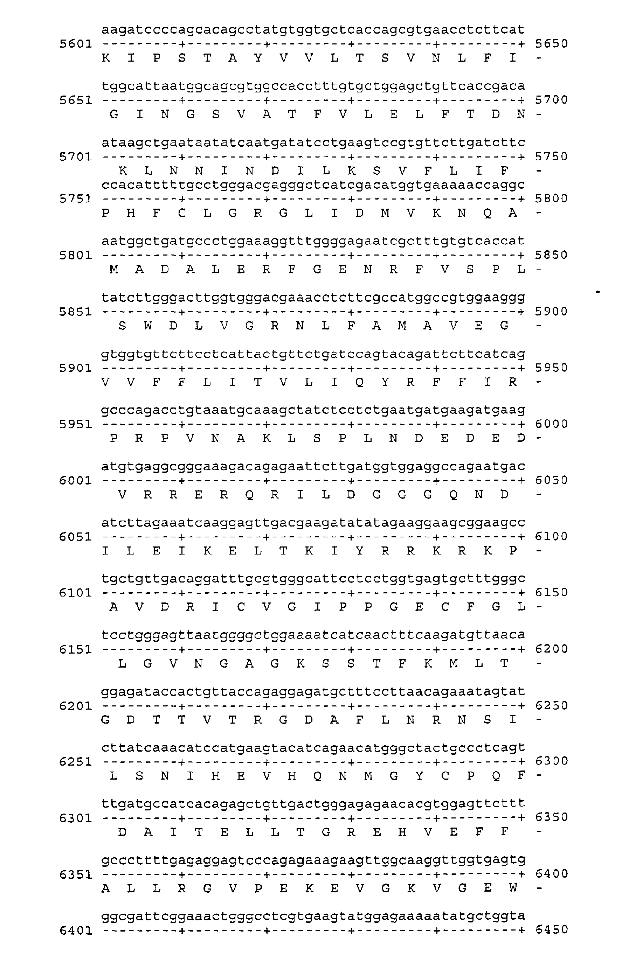

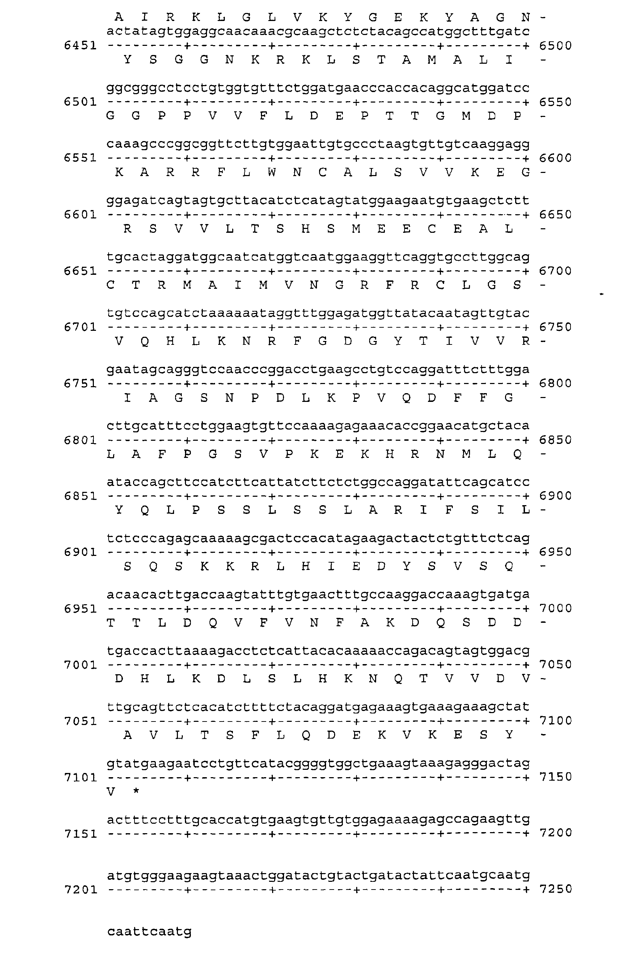

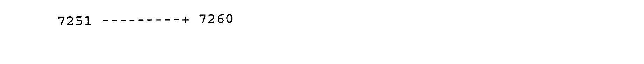

This invention provides the four polymorphisms G596A, T1136C, A2589G, and G3456C in the *ABC1* gene (*ABC1* gene see Figures 1 and 2), which affect the primary structure of the gene product. The frequencies of these *ABC1* polymorphisms were determined in a young and healthy population (Example 1).

The two most common polymorphisms (G596A and A2589G) are both associated with a decreased in vitro ApoA-I mediated efflux of cholesterol from mononuclear phagocytes, which is a typical feature of Tangier disease (Example 2).

No association of any of the polymorphisms were found with HDL-cholesterol, ApoA-I or any other lipoprotein fraction probably because the effect of the *ABC1* polymorphisms on the protein function is compensated by other factors controlling plasma HDL levels (Example 2 and 4)

The precise mechanism by which ABC1 promotes cellular lipid efflux is not completely defined. However, recently we could show that ABC1 is expressed on the plasma membrane and the Golgi complex, mediates ApoA-I associated export of cholesterol and phospholipids from the cell, and is regulated by cholesterol flux. Also, our results indicate that ABC1 is involved in lipid export processes involving vesicular budding between the Golgi and the plasma membrane. In addition to a possible function as direct lipid transporter, ABC1 may also be involved in the initiation of signal transduction processes stimulating intracellular lipid transport pathways (40). The finding that pretreatment of monocytes with forskolin, an cAMP elevating agent known to stimulate ApoA-I dependent lipid efflux (28), was able to normalize cholesterol and phospholipid efflux in 596A/A and 2589G/G subjects, may indicate that these gene variants affect the ABC1 function, which is related both to signaling and lipid transport. Since such a defect could be overcome by regulatory signals, this hypothesis may also provide a possible explanation for the lack of an effect on serum HDL levels.

In summary, these data emphasizes the role of ABC1 in lipid efflux, the first step in reverse cholesterol transport, and can be taken as evidence that common polymorphisms in *ABC1* directly affect cellular lipid homeostasis, which is a key factor in the atherogenetic process.

### Frequencies of the identified ABC1 gene polymorphisms in men with low HDL cholesterol levels and coronary heart disease

The frequencies of three of the common single nucleotide polymorphisms in *ABC1* (G596A, A2589G, and G3456C) were found to be significantly increased in the population comprised of men having HDL deficiency and established CHD (participants enrolled in the Veterans Affairs Cooperative HDL Cholesterol Intervention Trial ,VA-HIT) relative to control subjects from the Framingham Offspring Study (FOS). Moreover, associations were observed between *ABC1* polymorphisms and status, indicating that wild type alleles were consistently more prevalent in FOS than in VA-HIT but that mutant alleles were consistently more prevalent in VA-HIT (Example 3).

In summary, these data demonstrate that three of the common variants in the gene encoding ABC1 occur with significantly increased frequency in a population of men having low HDL-C as their primary lipid abnormality and established CHD. These results indicate that common mutations in the *ABC1* play a role in the development of CHD in the general population. These data also provide evidence for a role of *ABC1* in the premature CHD associated with common HDL deficiency states. All three *ABC1* variants are either conserved between human and mouse or are very similar as in the case of A2589G (isoleucine and valine, respectively), suggesting that each has an important functional role.

Furthermore, the identification of ABC1 as a transporter for IL-1β identifies this gene as a candidate gene for treatment of inflammatory diseases including rheumatoid arthritis and septic shock (27). The cytokine IL-1β is a broadly acting proinflammatory mediator that has been implicated in the pathogenesis of these diseases.

Moreover, we could demonstrate, that glyburide as an inhibitor of IL-1β secretion inhibits not only Caspase I mediated processing of pro-IL-1β and release of mature IL-1β but simultaneously inhibits ceramide formation from sphingomyelin mediated by neutral sphingomyelinase and thereby releases human fibroblasts from G₂-phase cell cycle arrest. These data provide a further mechanism indicative for a function of ABC1 in signalling and cellular lipid metabolism.

Autoimmune disorders that are associated with the antiphospholipid syndrome (e.g. lupus erythematodes) can be related to dysregulation of B-cell and T-cell function, aberrant antigen processing, or aberrations in the asymmetric distribution of membrane phospholipids. ABC-transporters are, besides their transport function, candidate genes for phospholipid translocases, floppases and scramblases that regulate phospholipid asymmetry (outer leaflet: PC+SPM; inner leaflet: PS+PE) of biological membranes [11]. There is considerable evidence for a dysregulation of members of the ABC-transporter family in patient cells. We conclude that these ABC-cassettes are also candidate genes for a genetic basis of antiphospholipid syndromes such as in Lupus erythematodes.

In summary, our findings emphasize how insight into the pathophysiology of a major disease process may be gleaned from a very rare genetic disorder.

Lipid disorders represent diseases characterized by changes in the lipid- and lipoproteinconcentrations in the organism. Cardiovascular diseases represent diseases of the heart and vessel system including coronary heart disease and atherosclerosis. Inflammatory diseases include diseases such as psoriasis and lupus erythematodes.

This invention is directed to the nucleotide sequences of the identified *ABC1* polymorphism variants, the corresponding protein sequences, antibodies, pharmaceutical compositions, diagnostic tests, vectors, and vector host systems according to the claims.

Our findings identify *ABC1* as a target for the diagnosis of lipid disorders, cardiovascular diseases , and inflammatory diseases.

Our findings further identify *ABC1* as a target for therapeutic strategies aimed at the prevention of lipid disorders, cardiovascular diseases, and inflammatory diseases.

The identified *ABC1* polymorphisms may create immunogenic protein sites that can be used for the generation of antibodies. These antibodies can be used for the detection of mutated ABC1-protein and can be used for the diagnosis of lipid disorders, cardiovascular diseases, and inflammatory diseases.

Therapeutic strategies also include the gene therapy of lipid disorders, cardiovascular diseases, and inflammatory diseases. For this gene therapy, a polynucleotid consisting of a transcription unit is used. The transcription unit consists of at least 9 consecutive nucleotides that are identical or substantial similiar to the ABC1 cDNA sequence. Alternatively, the transcription unit consists of at least 9 consecutive nucleotides leading to the production of antisense RNA of wild type ABC1 or ABC1 containing one or more of the identified polymorphisms. The transcription unit is combined with heterologous transcription regulating sequences, which regulate the transcription in human cells. The heterologous transcription regulating sequences include a promoter that is constitutively active in human cells. Alternatively, the heterologous transcription regulating sequences can contain a promoter that can be induced or repressed in human cells by the addition of a regulatory substance.

Another therapeutic approach would be the application of antisense molecules or ribozymes or RNA decoys directed towards the *ABC1* transcripts resulting in a modulation of the ABC1 expression. The methodology has been reviewed in (41).

This invention also includes a vector containing the above described transcription unit and transcription regulating sequences. Preferred vector is a virus, e.g. retrovirus, adenovirus, adeno-associated virus, herpes simplex virus, lentivirus, vaccinia virus, sindbis virus, semliki forest virus.

Preferred vectors are also plasmids.

The invention also includes a pharmaceutical preparation that contains the recombinant vectors, e.g. in a colloidal dispersion system. Preferred colloidal dispersion systems are liposomes or polylysin ligands.

The invention also includes pharmaceutical compositions that contain the above described recombinant vector constructs in combination with nontoxic, inert, pharmaceutically-suited carrier substances. Application of these pharmaceutical compositions can be locally or systemically (e.g. intravenous, intraarterious, intramuscular, subcutane, intradermal, anal, vaginal, nasal, transdermal, intraperitoneal, oral, or as aerosol).

The invention further includes recombinant host cells, especially recombinant eucaryotic host cells, that contain the above described recombinant vector constructs.

### Examples

### Example 1

### ATP-binding cassette transporter 1 (ABC1) gene polymorphisms

### Complete cDNA sequence of human ABC1

We have cloned the complete cDNA sequence of human *ABC1* (10). The nucleotide sequence of the complete coding region of the human ABC1 gene is shown in Fig. 1. The open reading frame of 6603 bp encodes a 2201 amino acid protein with a predicted molecular weight of 220 kDa. The protein sequence of the human ABC1 gene is shown in Fig. 2.

### Identification of four polymorphisms in human ABC1

Complete sequence analysis (by dye primer sequencing ?) of human *ABC1* in different (how many ?)Tangier kindred's revealed several polymorphisms, which were also found in healthy control subjects. These are G596A, T1136C, A2589G, and G3456C. All of them change the amino acid sequence of ABC1 and therefore may affect the function of this transporter.

### Frequency of polymorphisms in the general population

In order to determine the frequency of these four polymorphisms in the general population, we have tested 516 healthy blood donors. Table 1 summarizes the characteristics of the blood donors. The allele frequencies shown in table 2 demonstrate that G596A, A2589G and G3456C variants are quite common in a young, healthy population. The G596A polymorphism had the highest prevalence with an estimated frequency of 29.8% for the minor allele (596A), followed by A2589G with 13.7% (2589G), and G3456C with 2.5% (3456C). There was no significant deviation from the Hardy-Weinberg equilibrium. Only only 3 heterozygotes were found for T1136C in our group.

When we further analyzed the distribution of the polymorphisms in healthy blood donors, we detected a frequent coincidence of the G596A and A2589G polymorphisms (table 3). In homozygote individuals for the minor G596A allele (596A/A; n=41) the frequency of the alleles A and G of the A2589G polymorphism was 73.2% and 26.8% respectively, while in homozygotes for the major G596A allele (596G/G; n=248) the frequencies were 91.3% and 8.7% (p < 0.001). This indicates that the two mutations are in linkage disequilibrium in our population. While there was apparently no linkage disequilibrium between G596A and G3456C, the numbers were too small to exclude this with certainty (data not shown).

### Methods

*Sample collection.* Study subjects were recruited from healthy blood donors at the Institute for Clinical Chemistry (department of transfusion medicine) at the University of Regensburg. Samples from 516 individuals who gave consent to genetic analysis at the time of recruitment were available for the study. At the day of sample collection lipid parameters were determined and DNA-extraction was performed. A local review committee approved the Sample collection for genetic analysis.

*DNA extraction.* All DNA samples were isolated from whole blood with a QIAamp DNA Blood Maxi Kit (Qiagen) according to the manufacturer's recommendations.

*Detection of polymorphisms.* Genotype analysis was performed on the LightCycler (Roche Diagnostics) as previously described, with minor modifications (29,30). The amplification primers and hybridization probes are listed in table 4. The latter are designated as detection or anchor probes. They are labeled with LightCycler-Red 640 or 705 and fluorescein, respectively. We performed 40 amplification cycles under the following conditions: denaturation at 95°C for 120 s in the first step and 0 s in all subsequent steps, annealing at 57°C for 10 s, extension at 72°C for 15 s. This was followed by a temperature gradient melting curve analysis as described elsewhere (29,30). All detection probes recognize wild-type sequences, with the exception of the probe DET1136/mut, which is complementary to the sequence of the minor allele, i.e. 1136C. In order to assure specificity and accuracy of the LightCycler analysis 100 randomly selected samples were subjected to automated sequencing (ABI Prism 310 Genetic Analyzer), which proved full concordance of the results.

*Statistical Analysis.* To compare allele frequencies we employed chi-square test.

### Example 2

### ABC1 gene polymorphisms are associated with impaired ApoA-I mediated cholesterol efflux but not with decreased serum HDL or ApoA-1

In Tangier patients, mutations in *ABC1* are associated with severely decreased HDL-cholesterol and a lack or marked reduction of ApoA-I induced cholesterol and phospholipid efflux. Therefore, we hypothesized that HDL-cholesterol and ApoA-I levels as well as cholesterol and phospholipid efflux might differ between homozygous wild-type genotypes and carriers of one of the polymorphisms. Since the T1136C polymorphism was only found in heterozygous form in three individuals we did not include T1136C in our statistical analysis.

### Analysis of the association of the identified ABC1 gene polymorphisms with HDL-cholesterol, ApoA-I, or other lipoprotein fractions

We first analyzed the distribution of lipid and lipoprotein values in individuals with different genotypes. As indicated in table 5 neither heterozygous nor homozygous carriers of the G596A, A2589G, or G3456C polymorphisms had significantly different HDL-cholesterol or ApoA-I levels compared to the homozygous wild type probands. We also analyzed further parameters of plasmatic lipoprotein metabolism, even though this was not an a priori hypothesis. Total plasma cholesterol, triglycerides, and LDL-cholesterol were also not significantly different among the various genotypes.

### Analysis of the association of the identified ABC1 gene polymorphisms with cholesterol and phospholipid efflux

We then analyzed cholesterol and phospholipid efflux from mononuclear phagocytes isolated from individuals homozygous for the wild type nucleotide at all four polymorphic sites and from individuals homozygous for the polymorphic allele at position 596 or 2589. Monocytes from subjects homozygous for at least one polymorphic allele were found to have a highly significant reduction in ApoA-I mediated cholesterol efflux (table 6 A). Whereas in cholesterol loaded monocytes from wild type individuals ApoA-I incubation for 17 hrs increases cholesterol efflux to approximately 130% of baseline values, no stimulation of efflux was observed in subjects homozygous for G596A (596A/A) or A2589G (2589G/G). This finding was independent of gender. In order to investigate, whether the lack of cholesterol efflux could be overcome by regulatory mechanisms, cells were pre-incubated with forskolin, a cAMP-elevating agent, which is known to stimulate ApoA-I mediated lipid efflux (28). As shown in table 6 B forskolin treatment normalized cholesterol efflux in subjects with polymorphic alleles.

A tendency towards reduced ApoA-I mediated efflux of choline containing phospholipids was also correlated with the presence of both variant alleles but did not reach statistical significance (table 6 C). Similar to the ApoA-I mediated cholesterol efflux, forskolin pretreatment abolished the differences between wild type and 596A/A or 2589G/G, respectively (table 6 D).

### Methods

*Lipids and Lipoproteins.* Venous blood was taken from all blood donors after a 12-h fast. The samples were obtained in polypropylene tubes, centrifuged at 4000 x g for 10 min. Cholesterol, triglycerides, and HDL-cholesterol was measured by standard clinical chemistry protocols as previously described. LDL cholesterol was calculated by the Friedewald equation. ApoA-I was determined on a BNA nephelometer (Dade-Behring) with reagents from Dade-Behring.

*Cellular Lipid Efflux.* Human peripheral blood cells were isolated from lithium-heparin treated blood samples of adult, healthy volunteers. The peripheral blood mononuclear cell fraction was separated by density gradient centrifugation over Ficoll-Histopaque 1.007 (Sigma). Cells positive for CD2, CD7, CD16, CD19 and CD56 were removed by appropriate magnetic beads (Dynal) leaving > 80% pure monocytes. Isolated monocytes were then cultured at a density of 1.5x10⁵ cells/mL in polystyrene culture dishes (100x15mm Petri dish, Falcon) in a serum-free macrophage medium (Macrophage-SFM, Gibco Life Technologies) supplemented with 50ng/mL human recombinant M-CSF (R&D Systems). After an overnight incubation cells were cholesterol loaded for 24 hrs by addition of enzymatically modified LDL (E-LDL; 40 µg/ml), which was prepared as previously described (31). The incubation medium was further supplemented with ¹⁴C-cholesterol (1.5 µCi/ml) and ³H-choline (10µCi/ml) in order to label cellular cholesterol and choline containing phospholipids. In a subset of experiments cells were stimulated with 10 µM forskolin during the labeling period. After 24 hrs monocytes were washed and incubated for 17 hrs with or without 10 µg/ml ApoA-I (Sigma) in macrophage medium containing 0.1 % bovine serum albumin and 50ng/mL M-CSF. After incubation the media were removed and centrifuged at 800 x g to precipitate any detached cells. Cells were lysed in 0.2% SDS. Lipids were extracted by liquid extraction according to the method of Bligh and Dyer (13). ³H and ¹⁴C radioactivities of the total lipid extracts were measured by liquid scintillation counting in order to distinguish between ³H labeled phospholipids and ¹⁴C-cholesterol. Lipid efflux was calculated as percentage of disintegrations per minute (dpm) in medium divided by the sum of dpm recovered from medium and cells. The ApoA-I specific efflux is expressed a percent of basal unstimulated efflux observed in serum-free macrophage medium without ApoA-I.

*Statistical Analysis.* For comparison of lipid parameters and efflux data between wild type and polymorphisms the two-sample t test was used.

### Example 3

### Frequencies of the identified ABC1 gene polymorphisms in men with low HDL cholesterol levels and coronary heart disease

To adress the question whether the identified polymorphisms in *ABC-1* can provide insight into common HDL deficiency states and CHD risk, we compared the frequencies of three common *ABC1* variants (G596A, A2589G, and G3456C) between two different populations.

One population consisted of men participating in the Framingham Offspring Study (FOS), a long-term prospective evaluation of cardiovascular disease risk factors (32), whereas the other population consisted of men enrolled in the Veterans Affairs Cooperative HDL Cholesterol Intervention Trial (VA-HIT). VA-HIT, a multicenter study initiated in 1991, was designed to address the hypothesis that increasing HDL-C levels with gemfibrozil therapy would reduce the incidence of death from CHD and nonfatal myocardial infarction in men with established CHD, who had reduced HDL-C concentrations (≤40mg/dL; 1 mmol/L) and normal LDL-C levels (33). The characteristics of these two groups are provided in table 7. Men participating in VA-HIT were older, and slightly heavier, than men in FOS. With respect to plasma lipids, men in VA-HIT had significantly lower concentrations of plasma total cholesterol (-15%), HDL-C (-27%), and LDL-C (-18%), with the greatest difference between the groups noted in HDL-C levels. These differences resulted in a TC:HDL-C ratio which was significantly increased in VA-HIT relative to FOS.in control subjects from the FOS with those of participants in the VA-HIT.

### Screening of the FOS and VA-HIT populations for the presence of the identified ABC1 polymorphisms

In order to test the hypothesis that *ABC1* polymorphisms may play a role in common HDL deficiency states and, ultimately, CHD risk, genomic DNA from 1,014 men per FOS and VA-HIT population, respectively, was screened for the presence of three common single nucleotide polymorphisms in the *ABC1* gene (G596A, A2589G, and G3456C), using sequence-specific primers with the LightCycler System. The frequency distribution of each *ABC1* polymorphism in the FOS and VA-HIT populations is provided in table 8. With regard to the G596A polymorphism, the frequency of the mutant A allele was significantly increased (P=0.000) in VA-HIT subjects (0.314) relative to FOS (0.266), translating into fewer wild type, but more heterozygous, individuals in the former group. This was similarly the case for the A2589G mutation in which the mutant G allele frequency was 0.162 in VA-HIT versus 0.122 in FOS (P<0.001). The G3456C polymorphism was rare in both the FOS and VA-HIT populations; however, a significant increase (P<0.032) in the frequency of the mutant C allele was observed in VA-HIT subjects (0.038 vs. 0.026). Thus, the frequency of each of these single nucleotide polymorphisms was significantly increased in a population comprised of men having HDL deficiency and established CHD.

### Analysis of the associations between each ABC1 polymorphism and status

We further evaluated these data for the presence of associations between each *ABC1* polymorphism and status. Associations between the G596A and A2589G, G596A and G3456C, and A2589G and G3456C *ABC1* variants in FOS and VA-HIT subjects are presented in tables 9-11, respectively. In each case, a significantly greater proportion of FOS subjects possessed a pair of wild type alleles relative to VA-HIT subjects. In contrast, a significantly greater proportion of VA-HIT subjects possessed either one or two mutant alleles for each *ABC1* variant. These data indicate that wild type alleles were consistently more prevalent in FOS than in VA-HIT, with mutant alleles consistently more prevalent in VA-HIT relative to FOS, suggesting that the occurrence of these *ABC1* variants may be linked.

### Methods

*Subjects.* Subjects were men participating in either the Framingham Offspring (n=1,014) or VA-HIT (n=1,014) studies. The design and methods for FOS have been described elsewhere in detail (32), as have the rationale, design, and results of the VA-HIT study (33). FOS subjects were participants in cycle 4 of FOS and were free of clinical evidence of CHD. For VA-HIT, men were recruited at 20 Veterans Affairs medical centers throughout the United States. Eligibility for the trial required a documented history of CHD, an age of <74 years, an absence of coexisting conditions, an HDL-C level of ≤40 mg/dL (1.0 mmol/L), an LDL-C of ≤140 mg/dL (3.6 mmol/L), and a triglyceride level of <300 mg/dL (3.4 mmol/L). Since DNA was available from 1,014 men participating in the VA-HIT study, an equal number of men participating in cycle 4 of FOS was randomly selected from a total of 1,139 samples available for analysis. Informed consent was obtained from all subjects. Nearly all of the subjects in both groups were Caucasians. Information on alcohol consumption, smoking, blood pressure, body mass index (BMI), and diabetes were available for all subjects enrolled in these studies. Plasma lipid and lipoprotein data used in our analyses were obtained from FOS subjects who were not taking a lipid-lowering medication, while those from the VA-HIT subjects were obtained at baseline, prior to randomization to a treatment (gemfibrozil, 600 mg bid) or placebo group.

*DNA Isolation.* Genomic DNA was extracted from whole blood samples using either QIAamp mini kits (Qiagen) or Generation Capture Column® kits (Gentra Systems).

***Mutation detection.*** Genotype analysis was performed using LightCycler technology (Roche Diagnostics), as described elsewhere in detail (28,34,35). Briefly, this method enables recognition of alterations in a nucleotide sequence through the detection of changes in fluorescence emission, dependent upon energy transfer between fluorophores on two adjacent hybridization probes. Both probes recognize the sequence of a specific amplicon. The detection probe, labeled on its 5' end with LightCycler-Red 640 or 705, overlaps the site of a polymorphic nucleotide, whereas the anchor probe, carrying fluorescein at the 3' end, binds to the amplicon 1 to 3 nucleotides upstream of the detection probe. After PCR in the presence of target DNA, samples are briefly denatured at 95°C, rapidly cooled to 35-40°C, and, in the final step, heated gradually to 70-80°C. Differentiation between a wild type and polymorphic allele is possible, because the detection probe will detach at a different melting temperature dependent upon the presence of a mismatch between the probe and amplicon sequences.

*Statistical analyses.* To compare the FOS and VA-HIT populations, we employed chi-square tests for categorical measures and two-sample t test for continuous measures, using the SYSTAT statistical package.

### Example 4

### Analysis of the associations between each ABC1 polymorphism and plasma lipid profiles in FOS

Analyses were also performed to test for potential associations between each *ABC1* polymorphism and plasma lipid profiles in FOS. Because the VA-HIT subjects were selected primarily on the basis of plasma lipid values and CHD, we did not test for associations in this group, which was, by design, more homogeneous than that of FOS. In FOS, no statistically significant relationships were detected between any of the 3 *ABC1* polymorphisms and plasma lipids (data not shown). This remained the case after adjustment for familial relationships, age, BMI, smoking, alcohol intake, and apolipoprotein E genotype. Although this result is somewhat incongruous with evidence for a role of ABC1 in the regulation of plasma HDL-C levels (9,11-15), we believe that *ABC1* polymorphisms may directly influence intracellular cholesterol trafficking and, ultimately, reverse cholesterol transport. Support for this hypothesis is provided by the results presented in *Example 2* demonstrating that the homozygosity for either the G596A or A2589G polymorphism is associated with decreased cellular cholesterol efflux in healthy, young subjects. Moreover, because HDL-C levels are modulated by other genetic and environmental factors (7, 36), direct effects of *ABC1* polymorphisms on plasma HDL-C concentrations may not be readily apparent, especially in the heterozygous state.

### Methods

*Measurement of plasma lipids and lipoproteins.* Blood samples were collected from subjects, after a 12-14 h fast, into tubes containing 0.1% EDTA. Plasma was isolated and frozen for subsequent analysis of plasma lipid and lipoprotein concentrations. Plasma total cholesterol and triglyceride concentrations were determined using enzymatic assays (37). Plasma HDL-C concentrations were measured after dextran sulfate-magnesium precipitation of apoB-containing lipoproteins (38), and LDL-C levels were calculated with the equation of Friedewald, Levy, and Fredrickson (39).

*Statistical analyses.* To evaluate the relationships between ABC1 genotype and plasma lipids, we used analysis of covariance techniques, employing several different models to adjust for potential confounders. Age, BMI, smoking status, alcohol consumption, and apolipoprotein E genotype were included in these models. Loglinear models were used to examine associations between the 3 polymorphisms and status (FOS or VA-HIT). For all 3 models, statistically significant interactions were detected between ABC1 genotype and status, indicating that differences existed between the FOS and VA-HIT groups with regard to the presence of mutant alleles. Specifically, for each ABC1 polymorphism, those in the FOS group were more likely to be categorized as wild type, whereas VA-HIT subjects were more likely to be classified as heterozygous.

## Claims

1. A polynucleotide comprising a member selected from the group consisting of:
(a) a polynucleotide as set forth in SEQ ID NO:1 with the sequence variants G596A or T1136C or A2589G or G3456C or any combination of these variants;
(b) a polynucleotide fragment of the polynucleotides of (a) containing the variants G596A or T1136C or A2589G or G3456C or any combination of these variants.

2. The polynucleotide of claim 1 wherein the polynucleotide is DNA.

3. A vector containing one or more of the polynucleotides of claim 1 and 2.

4. A host cell containing the vector of claim 3.

5. A process for producing a polypeptide comprising: expressing from the host cell of claim 4 the polypeptide encoded by said DNA.

6. An assay using one of the materials of claims 1 to 4 for the detection of the ABC1 gene transcripts.

7. An assay using one of the materials of claims 1 to 4 for the detection of the ABC1 variants G596A or T1136C or A2589G or G3456C.

8. A polypeptide selected from the group consisting of:
a) a polypeptide having the deduced amino acid sequence of SEQ ID NO:1 and
b) fragments, analogs and derivatives thereof containing the sequence variants of claim 2b.

9. An antibody capable to bind to the polypeptide of claim 8.

10. An assay using one of the materials of claims 8 and 9 for the detection of
(a) the ABC1 protein or fragments, analogs and derivatives thereof,
(b) the deduced protein or fragments, analogs and derivatives thereof from the ABC1 sequence variants G596A or T1136C or A2589G or G3456C .

11. A diagnostic kit for the detection of the ABC1 gene variants G596A or T1136C or A2589G or G3456C.

12. Modulator of polypeptides encoded by a polynucleotide comprising a member selected from the group consisting of:
(a) a polynucleotide as set forth in SEQ ID NO:1 and
(b) a polynucleotide fragment of the polynucleotide of (a) containing the sequence variants G596A or T1136C or A2589G or G3456C.

13. A pharmaceutical comprising the modulator of claim 12.

14. The modulation of ABC1 transcripts or proteins or fragments thereof by gene therapy.

15. The modulation of ABC1 transcripts or proteins or fragments thereof by antisense or ribozyme technology or RNA decoys.


SEQUENCE LISTING
<110> Bayer AG

<120> ATP binding cassette transporter 1 (ABC1) gene
      polymorphisms and uses thereof for the diagnosis
      and treatment of lipid disorders, cardiovascular
      diseases and inflammatory diseases

<130> ATP binding cassette transporter 1 (ABC1) gene

<140>
<141>

<150> 101706
<151> 1998-09-25

<160> 2

<170> PatentIn Ver. 2.0

<210> 1
<211> 6880
<212> DNA
<213> Human

<220>
<223> cDNA of ABCA1 (ABC1)

<400> 1
caaacatgtc agctgttact ggaagtggcc tggcctctat ttatcttcct gatcctgatc 60
tctgttcggc tgagctaccc accctatgaa caacatgaat gccattttcc aaataaagcc 120
atgccctctg caggaacact tccttgggtt caggggatta tctgtaatgc caacaacccc 180
tgtttccgtt acccgactcc tggggaggct cccggagttg ttggaaactt taacaaatcc 240
attgtggctc gcctgttctc agatgctcgg aggcttcttt tatacagcca gaaagacacc 300
agcatgaagg acatgcgcaa agttctgaga acattacagc agatcaagaa atccagctca 360
aacttgaagc ttcaagattt cctggtggac aatgaaacct tctctgggtt cctgtatcac 420
aacctctctc tcccaaagtc tactgtggac aagatgctga gggctgatgt cattctccac 480
aaggtatttt tgcaaggcta ccagttacat ttgacaagtc tgtgcaatgg atcaaaatca 540
gaagagatga ttcaacttgg tgaccaagaa gtttctgagc tttgtggcct accaagggag 600
aaactggctg cagcagagcg agtacttcgt tccaacatgg acatcctgaa gccaatcctg 660
agaacactaa actctacatc tcccttcccg agcaaggagc tggccgaagc cacaaaaaca 720
ttgctgcata gtcttgggac tctggcccag gagctgttca gcatgagaag ctggagtgac 780
atgcgacagg aggtgatgtt tctgaccaat gtgaacagct ccagctcctc cacccaaatc 840
taccaggctg tgtctcgtat tgtctgcggg catcccgagg gaggggggct gaagatcaag 900
tctctcaact ggtatgagga caacaactac aaagccctct ttggaggcaa tggcactgag 960
gaagatgctg aaaccttcta tgacaactct acaactcctt actgcaatga tttgatgaag 1020
aatttggagt ctagtcctct ttcccgcatt atctggaaag ctctgaagcc gctgctcgtt 1080
gggaagatcc tgtatacacc tgacactcca gccacaaggc aggtcatggc tgaggtgaac 1140
aagaccttcc aggaactggc tgtgttccat gatctggaag gcatgtggga ggaactcagc 1200
cccaagatct ggaccttcat ggagaacagc caagaaatgg accttgtccg gatgctgttg 1260
gacagcaggg acaatgacca cttttgggaa cagcagttgg atggcttaga ttggacagcc 1320
caagacatcg tggcgttttt ggccaagcac ccagaggatg tccagtccag taatggttct 1380
gtgtacacct ggagagaagc tttcaacgag actaaccagg caatccggac catatctcgc 1440
ttcatggagt gtgtcaacct gaacaagcta gaacccatag caacagaagt ctggctcatc 1500
aacaagtcca tggagctgct ggatgagagg aagttctggg ctggtattgt gttcactgga 1560
attactccag gcagcattga gctgccccat catgtcaagt acaagatccg aatggacatt 1620
gacaatgtgg agaggacaaa taaaatcaag gatgggtact gggaccctgg tcctcgagct 1680
gacccctttg aggacatgcg gtacgtctgg gggggcttcg cctacttgca ggatgtggtg 1740
gagcaggcaa tcatcagggt gctgacgggc accgagaaga aaactggtgt ctatatgcaa 1800
cagatgccct atccctgtta cgttgatgac atctttctgc gggtgatgag ccggtcaatg 1860
cccctcttca tgacgctggc ctggatttac tcagtggctg tgatcatcaa gggcatcgtg 1920
tatgagaagg aggcacggct gaaagagacc atgcggatca tgggcctgga caacagcatc 1980
ctctggttta gctggttcat tagtagcctc attcctcttc ttgtgagcgc tggcctgcta 2040

gtggtcatcc tgaagttagg aaacctgctg ccctacagtg atcccagcgt ggtgtttgtc 2100
ttcctgtccg tgtttgctgt ggtgacaatc ctgcagtgct tcctgattag cacactcttc 2160
tccagagcca acctggcagc agcctgtggg ggcatcatct acttcacgct gtacctgccc 2220
tacgtcctgt gtgtggcatg gcaggactac gtgggcttca cactcaagat cttcgctagc 2280
ctgctgtctc ctgtggcttt tgggtttggc tgtgagtact ttgccctttt tgaggagcag 2340
ggcattggag tgcagtggga caacctgttt gagagtcctg tggaggaaga tggcttcaat 2400
ctcaccactt cggtctccat gatgctgttt gacaccttcc tctatggggt gatgacctgg 2460
tacattgagg ctgtctttcc aggccagtac ggaattccca ggccctggta ttttccttgc 2520
accaagtcct actggtttgg cgaggaaagt gatgagaaga gccaccctgg ttccaaccag 2580
aagagaatat cagaaatctg catggaggag gaacccaccc acttgaagct gggcgtgtcc 2640
attcagaacc tggtaaaagt ctaccgagat gggatgaagg tggctgtcga tggcctggca 2700
ctgaattttt atgaggccag gatcacctcc ttcctgggcc acaatggagc ggggaagacg 2760
accaccatgt caatcctgac cgggttgttc cccccgacct cgggcaccgc ctacatcctg 2820
ggaaaagaca ttcgctctga gatgagcacc atccggcaga acctgggggt ctgtccccag 2880
cataacgtgc tgtttgacat gctgactgtc gaagaacaca tctggttcta tgcccgcttg 2940
aaagggctct ctgagaagca cgtgaaggcg gagatggagc agatggccct ggatgttggt 3000
ttgccatcaa gcaagctgaa aagcaaaaca agccagctgt caggtggaat gcagagaaag 3060
ctatctgtgg ccttggcctt tgtcggggga tctaaggttg tcattctgga tgaacccaca 3120
gctggtgtgg acccttactc ccgcagggga atatgggagc tgctgctgaa ataccgacaa 3180
ggccgcacca ttattctctc tacacaccac atggatgaag cggacgtcct gggggacagg 3240
attgccatca tctcccatgg gaagctgtgc tgtgtgggct cctccctgtt tctgaagaac 3300
cagctgggaa caggctacta cctgaccttg gtcaagaaag atgtggaatc ctccctcagt 3360
tcctgcagaa acagtagtag cactgtgtca tacctgaaaa aggaggacag tgtttctcag 3420
agcagttctg atgctggcct gggcagcgac catgagagtg acacgctgac catcgatgtc 3480
tctgctatct ccaacctcat caggaagcat gtgtctgaag cccggctggt ggaagacata 3540
gggcatgagc tgacctatgt gctgccatat gaagctgcta aggagggagc ctttgtggaa 3600
ctctttcatg agattgatga ccggctctca gacctgggca tttctagtta tggcatctca 3660
gagacgaccc tggaagaaat attcctcaag gtggccgaag agagtggggt ggatgctgag 3720
acctcagatg gtaccttgcc agcaagacga aacaggcggg ccttcgggga caagcagagc 3780
tgtcttcgcc cgttcactga agatgatgct gctgatccaa atgattctga catagaccca 3840
gaatccagag agacagactt gctcagtggg atggatggca aagggtccta ccaggtgaaa 3900
ggctggaaac ttacacagca acagtttgtg gcccttttgt ggaagagact gctaattgcc 3960
agacggagtc ggaaaggatt ttttgctcag attgtcttgc cagctgtgtt tgtctgcatt 4020
gcccttgtgt tcagcctgat cgtgccaccc tttggcaagt accccagcct ggaacttcag 4080
ccctggatgt acaacgaaca gtacacattt gtcagcaatg atgctcctga ggacacggga 4140
accctggaac tcttaaacgc cctcaccaaa gaccctggct tcgggacccg ctgtatggaa 4200
ggaaacccaa tcccagacac gccctgccag gcaggggagg aagagtggac cactgcccca 4260
gttccccaga ccatcatgga cctcttccag aatgggaact ggacaatgca gaacccttca 4320
cctgcatgcc agtgtagcag cgacaaaatc aagaagatgc tgcctgtgtg tcccccaggg 4380
gcaggggggc tgcctcctcc acaaagaaaa caaaacactg cagatatcct tcaggacctg 4440
acaggaagaa acatttcgga ttatctggtg aagacgtatg tgcagatcat agccaaaagc 4500
ttaaagaaca agatctgggt gaatgagttt aggtatggcg gcttttccct gggtgtcagt 4560
aatactcaag cacttcctcc gagtcaagaa gttaatgatg ccaccaaaca aatgaagaaa 4620
cacctaaagc tggccaagga cagttctgca gatcgatttc tcaacagctt gggaagattt 4680
atgacaggac tggacaccag aaataatgtc aaggtgtggt tcaataacaa gggctggcat 4740
gcaatcagct ctttcctgaa tgtcatcaac aatgccattc tccgggccaa cctgcaaaag 4800
ggagagaacc ctagccatta tggaattact gctttcaatc atcccctgaa tctcaccaag 4860
cagcagctct cagaggtggc tccgatgacc acatcagtgg atgtccttgt gtccatctgt 4920
gtcatctttg caatgtcctt cgtcccagcc agctttgtcg tattcctgat ccaggagcgg 4980
gtcagcaaag caaaacacct gcagttcatc agtggagtga agcctgtcat ctactggctc 5040
tctaattttg tctgggatat gtgcaattac gttgtccctg ccacactggt cattatcatc 5100
ttcatctgct tccagcagaa gtcctatgtg tcctccacca atctgcctgt gctagccctt 5160
ctacttttgc tgtatgggtg gtcaatcaca cctctcatgt acccagcctc ctttgtgttc 5220
aagatcccca gcacagccta tgtggtgctc accagcgtga acctcttcat tggcattaat 5280
ggcagcgtgg ccacctttgt gctggagctg ttcaccgaca ataagctgaa taatatcaat 5340
gatatcctga agtccgtgtt cttgatcttc ccacattttt gcctgggacg agggctcatc 5400
gacatggtga aaaaccaggc aatggctgat gccctggaaa ggtttgggga gaatcgcttt 5460
gtgtcaccat tatcttggga cttggtggga cgaaacctct tcgccatggc cgtggaaggg 5520
gtggtgttct tcctcattac tgttctgatc cagtacagat tcttcatcag gcccagacct 5580
gtaaatgcaa agctatctcc tctgaatgat gaagatgaag atgtgaggcg ggaaagacag 5640
agaattcttg atggtggagg ccagaatgac atcttagaaa tcaaggagtt gacgaagata 5700
tatagaagga agcggaagcc tgctgttgac aggatttgcg tgggcattcc tcctggtgag 5760
tgctttgggc tcctgggagt taatggggct ggaaaatcat caactttcaa gatgttaaca 5820

ggagatacca ctgttaccag aggagatgct ttccttaaca gaaatagtat cttatcaaac 5880
atccatgaag tacatcagaa catgggctac tgccctcagt ttgatgccat cacagagctg 5940
ttgactggga gagaacacgt ggagttcttt gcccttttga gaggagtccc agagaaagaa 6000
gttggcaagg ttggtgagtg ggcgattcgg aaactgggcc tcgtgaagta tggagaaaaa 6060
tatgctggta actatagtgg aggcaacaaa cgcaagctct ctacagccat ggctttgatc 6120
ggcgggcctc ctgtggtgtt tctggatgaa cccaccacag gcatggatcc caaagcccgg 6180
cggttcttgt ggaattgtgc cctaagtgtt gtcaaggagg ggagatcagt agtgcttaca 6240
tctcatagta tggaagaatg tgaagctctt tgcactagga tggcaatcat ggtcaatgga 6300
aggttcaggt gccttggcag tgtccagcat ctaaaaaata ggtttggaga tggttataca 6360
atagttgtac gaatagcagg gtccaacccg gacctgaagc ctgtccagga tttctttgga 6420
cttgcatttc ctggaagtgt tccaaaagag aaacaccgga acatgctaca ataccagctt 6480
ccatcttcat tatcttctct ggccaggata ttcagcatcc tctcccagag caaaaagcga 6540
ctccacatag aagactactc tgtttctcag acaacacttg accaagtatt tgtgaacttt 6600
gccaaggacc aaagtgatga tgaccactta aaagacctct cattacacaa aaaccagaca 6660
gtagtggacg ttgcagttct cacatctttt ctacaggatg agaaagtgaa agaaagctat 6720
gtatgaagaa tcctgttcat acggggtggc tgaaagtaaa gagggactag actttccttt 6780
gcaccatgtg aagtgttgtg gagaaaagag ccagaagttg atgtgggaag aagtaaactg 6840
gatactgtac tgatactatt caatgcaatg caattcaatg 6880

<210> 2
<211> 2201
<212> PRT
<213> Human

<220>
<223> Peptide sequence of ABCA1 (ABC1)

<400> 2